# EUROPÄISCHE PATENTSCHRIFT

(11) **EP 4 608 344 B1**
(45) Veröffentlichungstag und Bekanntmachung des Hinweises auf die Patenterteilung: **23.09.2026**
(21) Anmeldenummer: 23798381.2
(22) Anmeldetag: 25.10.2023
(51) Int. Cl.: A61F 5/30, A61F 5/01

(54) **PELOTTE MIT DEHNUNGSBEREICHEN**
PAD WITH EXPANSION AREAS
PELOTE AVEC ZONES D'EXPANSION

(30) Priorität: 28.10.2022 DE 102022128741
(43) Veröffentlichungstag der Anmeldung: 03.09.2025
(73) Patentinhaber: Bauerfeind AG, 07937 Zeulenroda-Triebes (DE)
(72) Erfinder: HESS, Heinrich, 07937 Zeulenroda-Triebes (DE); WIEDNER, Juliane, 07937 Zeulenroda-Triebes (DE); BAUERFEIND-JOHNSON, Beatrix, 07937 Zeulenroda-Triebes (DE)
(74) Vertreter: Gleiss Große Schrell und Partner mbB
(86) Internationale Anmeldenummer: PCT/EP2023/079764
(87) Internationale Veröffentlichungsnummer: WO 2024/089091

(56) Entgegenhaltungen:
- EP-A1- 0 598 291
- EP-A1- 1 634 555
- DE-A1- 10 229 510
- DE-A1- 102012 105 626
- DE-U1- 20 005 663
- DE-U1- 202022 100 765

## Beschreibung

Die vorliegende Erfindung betrifft eine Pelotte für orthopädische Hilfsmittel, aufweisend einen Pelottengrundkörper mit mindestens zwei paarig angeordneten Dehnungsbereichen.

Weiterhin betrifft die Erfindung ein orthopädisches Hilfsmittel, insbesondere eine Orthese oder Bandage, die zumindest eine derartige Pelotte aufweist.

Pelotten, die für verschiedene prophylaktische und therapeutische Anwendungen eingesetzt werden, sind aus dem Stand der Technik bereits bekannt, wie zum Beispiel aus den Offenlegungsschriften DE 27 22 563 C2, der EP 0 598 291 A1 und der EP 0 600 218 A2.

Viele Patienten mit Kniearthrose leiden unter einer Retro-Patellararthrose mit Schmerzen bei Druck auf die Patella und dann auf das Gleitlager. Schon ein mäßiger, aber anhaltender Druck kann zu Schmerzen führen, wie zum Beispiel beim Tragen enger Jeanshosen. Die führende Muskulatur um die großen arthrotischen Gelenke ist dabei oft schmerzhaft verhärtet sowie verkürzt und es treten auf der Muskeloberfläche häufig schmerzhafte Triggerpunktverhärtungen auf. Bei einer Arthrose im Knie muss das Gewebe, der Gelenkspalt und die retropatellare Gegend entspannt werden. In der Physiotherapie werden dafür spezielle gezielte Massagetechniken angewandt. Zusätzlich oder alternativ werden Bandagen und Orthesen mit Pelotten verwendet, die durch Erhebungen, als Pelottendruckelemente wie beispielsweise Noppen, bei Druck auf den Muskel eine Massage mit Lösung der Kontrakturen und eine sensomotorische Antwort des Muskels erzeugen. Pelotten mit solchen Erhebungen, insbesondere Noppen zur Massage sind nicht nur für die Patella, sondern auch für andere Bereiche des Körpers bekannt und beispielsweise in der DE 42 38 610 A1 und der DE 10 2012 105 626 A1 beschrieben.

Die Noppen wirken dabei über den Muskeln, beispielsweise dem "Musculus vastus medialis" im Sinne der Sensomotorik. Im Ruhezustand ist die Druckwirkung/Kompression nur durch die Spannung des Gestricks der Bandage oder der Orthese bedingt. Bei Anspannung, also einer Kontraktion des Muskels kommt es zu einer Druckerhöhung infolge der Volumenvergrößerung des Muskels, wodurch eine sensomotorische Antwort erzeugt wird, die schmerzlindernd ist. Auch bei Bewegung, beispielsweise im Gehen durch die Knie- und Hüftbeugung, entsteht bei Belastung eine Druckerhöhung durch die Noppen.

Die DE 20 2022 100 765 U1, die den Oberbegriff des Anspruchs 1 zeigt, offenbart Pelotten mit Erhebungen und Nuten.

Bei Pelotten aus dem Stand der Technik, beispielsweise Patellapelotten, ist nachteilig, dass die Bewegung zum Verrutschen der Pelotte führt. Beispielsweise kann die Beugung bei einer Patellapelotte je nach Dehnungswiderstand der teripatellaren Pelotte, die häufig aus Silikon besteht, einen deutlichen distalen Zug auf den oberen Bandagenteil ausüben, was zu einer Rutschtendenz und bei einem Arthrotiker zu einem unangenehmen Druck auf die Patella führt. Gleichzeitig wird durch den Distalzug der senkrechte therapeutisch wirksame Noppendruck verringert, unter anderem auch weil die Noppen sich verbiegen und damit die erwünschte sensomotorische Antwort des Muskels vastus medialis erheblich abgeschwächt wird.

Der Erfindung liegt somit die Aufgabe zugrunde, eine Pelotte zu schaffen, die trotz vorteilhafter Massagefunktion ein Verrutschen der Pelotte bei Bewegung vermindert.

Der Erfindung liegt somit auch die Aufgabe zugrunde, eine Pelotte zu schaffen, die trotz vorteilhafter Massagefunktion und -wirkung weniger hoch aufbaut und einen angenehmen Tragekomfort auch bei längeren Anwendungen gewährleistet.

Die vorliegende Erfindung löst das ihr zugrunde liegende technische Problem durch eine Pelotte nach Anspruch 1.

Die vorliegende Erfindung löst das ihr zugrunde liegende technische Problem durch eine Pelotte, aufweisend einen Pelottengrundkörper, wobei der Pelottengrundkörper eine Vorderseite und eine Rückseite aufweist, wobei die Vorderseite des Pelottengrundkörpers mindestens eine Erhebung aufweist, wobei der Pelottengrundkörper mindestens zwei paarig angeordnete Dehnungsbereiche aufweist, wobei die zwei Dehnungsbereiche den Pelottengrundkörper in einen ersten Pelottengrundkörperbereich und einen zweiten Pelottengrundkörperbereich aufteilen und wobei es sich bei den mindestens zwei Dehnungsbereichen jeweils um mindestens eine Nut im Pelottengrundkörper handelt, wobei der Pelottengrundkörper eine Aussparung aufweist, wobei der Pelottengrundkörper eine Außenseite aufweist und eine zur Aussparung hin gerichtete Innenseite aufweist, wobei die mindestens eine Nut an der Außenseite beginnt und nicht bis zur Innenseite reicht oder an der Innenseite beginnt und nicht bis zur Außenseite reicht.

Bei der mindestens einen Nut handelt es sich also um eine Dehnungsnut, die in vorteilhafter Weise eine Dehnung im Dehnungsbereich des Pelottengrundkörpers, insbesondere zwischen dem ersten Pelottengrundkörperbereich und dem zweiten Pelottengrundkörperbereich ermöglichen.

Die paarige Anordnung der Dehnungsbereiche ermöglicht in vorteilhafter Weise eine gleichmäßige Dehnung. Bevorzugt ist bei der paarigen Anordnung der erste Dehnungsbereich an einem Randbereich des Pelottengrundkörpers positioniert und der zweite Dehnungsbereich an den gegenüberliegenden Randbereich des Pelottengrundkörpers positioniert. Die paarige Anordnung ist besonders vorteilhaft, wenn der Pelottengrundkörper eine Aussparung aufweist. Solche Aussparungen sind beispielsweise bei Patellapelotten oder Ellenbogenpelotten vorhanden. Diese Aussparungen sind dem Fachmann allgemein bekannt und dienen der Aufnahme der Patella oder des Ellenbogens, so dass der Pelottengrundkörper die Patella oder den Ellenbogen umfasst.

Erfindungsgemäß weist der Pelottengrundkörper eine Aussparung auf.

In eine bevorzugten Ausführungsform weist der Pelottengrundkörper mindestens zwei Dehnungsbereiche auf, die im Pelottengrundkörper durch die Aussparung getrennt werden. Bevorzugt liegen die beiden paarigen Dehnungsbereiche also neben der Aussparung, beispielsweise links und rechts von der Aussparung oder oberhalb und unterhalb der Aussparung. Bevorzugt weist die Aussparung einen Durchmesser von mindestens 3 cm auf, ist also insbesondere eine Aussparung, die die Patella, den Ellenbogen, also den Kopf der Elle oder den Kopf der Elle oder Speiche am Handgelenk umfassen kann. Größe und Form solcher Aussparungen sind dem Fachmann bekannt. Solche Aussparungen umrahmen meist knöcherne oder Knorpel- Vorsprünge am Körper (Protrusionen), so dass sich daraus Die Größe der Aussparungen ergibt.

Bevorzugt ist eine Pelotte, insbesondere Patellapelotte, Ellenbogenpelotte oder Handgelenkspelotte mit exakt zwei Dehnungsbereichen.

In einer besonders bevorzugten Ausführungsform befindet sich die mindestens eine Nut auf der Vorderseite des Pelottengrundkörpers. Erfindungsgemäß weist der Pelottengrundkörper eine Außenseite auf und eine zur Aussparung hin gerichtete Innenseite auf, wobei die mindestens eine Nut an der Außenseite beginnt und nicht bis zur Innenseite reicht oder an der Innenseite beginnt und nicht bis zur Außenseite reicht.

Somit handelt es sich bei der Nut, also Dehnungsnut nicht um durchgehende Kanäle mit zwei seitlichen Öffnungen, sondern um Einschnitte, die nur eine seitliche Öffnung haben oder keine seitliche Öffnung haben.

Bevorzugt hat die mindestens eine Nut eines Dehnungsbereichs, also Dehnungsnut, nur eine seitliche Öffnung oder keine seitliche Öffnung. Bevorzugt hat die mindestens eine Nut eines Dehnungsbereichs, also Dehnungsnut, nur eine seitliche Öffnung.

Bevorzugt besteht ein Dehnungsbereich aus mindestens einer Nut, beispielsweise einer Nut, zwei Nuten, drei Nuten, vier Nuten, fünf Nuten oder mehr Nuten.

Bevorzugt haben die beiden paarige angeordneten Dehnungsbereiche die gleiche Anzahl von Nuten und/oder von Form und Größe gleich ausgestaltete Nuten.

Durch die Anzahl, Breite und Tiefe der Nuten, also Dehnungsnuten wird die Dehnbarkeit beeinflusst.

Vorzugsweise weist die Nut, also Dehnungsnut eine Breite von mindestens 0,1 Millimeter auf. Vorzugsweise weist die Nut, also Dehnungsnut eine Breite von mindestens 0,3 Millimeter auf. Vorzugsweise weist die Nut, also Dehnungsnut eine Breite von 0,1 Millimeter bis 100 Millimeter, insbesondere von 0,3 Millimeter bis 50 Millimeter auf. Es hat sich gezeigt, dass hierdurch die oben genannten Eigenschaften in besonders vorteilhafter Weise erreicht werden. Vorzugsweise weist die Nut, also Dehnungsnut eine Tiefe von 0,1 Millimeter bis 10 Millimeter, insbesondere von 0,5 Millimeter bis 3 Millimeter auf. Es hat sich gezeigt, dass hierdurch die oben genannten Eigenschaften in besonders vorteilhafter Weise erreicht werden.

Gemäß einer bevorzugten Weiterbildung der Erfindung weist die Nut, also Dehnungsnut in ihrer Längserstreckung eine konstante Tiefe oder zumindest zwei unterschiedliche Tiefen auf.

Gemäß einer bevorzugten Ausführungsform der Erfindung weist die Nut, also Dehnungsnut eine Tiefe auf, die mindestens 10% der Höhe des Grundkörpers am Rand der Nut entspricht. Die Nut kann vergleichbar einer "Schallplattenrille" ausgestaltet sein. Somit kann diese in Ihrer Breite und ihrer Tiefe variabel ausgeprägt sein.

In einer bevorzugten Ausführungsform handelt es sich bei der mindestens einen Erhebung um ein Pelottendruckelement. In einer bevorzugten Ausführungsform handelt es sich bei der mindestens einen Erhebung um Noppen und/oder Stege. Geeignete Erhebungen sind dem Fachmann bekannt. Die Pelotte kann auch mehrere Erhebungen, insbesondere unterschiedliche Erhebungen aufweisen, die unterschiedlich auf der Vorderseite der Pelotte positioniert sind.

Die Erhebungen können aus demselben Material wie der Pelottengrundkörper gebildet sein oder aus einem anderen Material. Bevorzugt sind die Erhebungen aus einem anderen Material gebildet.

In einer bevorzugten Ausführungsform sind die Erhebungen härter als der Pelottengrundkörper.

In dieser Variante ist die Beweglichkeit und damit die Massagefunktion der Pelotte auch durch die Wahl der Materialien eingestellt. Insbesondere sind der Grundkörper und/oder das Erhebung, insbesondere Pelottendruckelement aus einem viskoelastischen Material gefertigt, das eine vorteilhafte Verformungen der Pelotte bei der Anwendung erlaubt, die zu dem gewünschten Massageeffekt führen. Insbesondere sind die Materialien derart gewählt, dass sie sich in Bezug auf ihre Steifigkeit oder Elastizität voneinander unterscheiden.

So ist gemäß einer bevorzugten ersten Ausführungsform vorgesehen, dass die Erhebung, insbesondere das Pelottendruckelement aus einem Material gefertigt ist, das härter als das Material des Grundkörpers ist. Dadurch können die Erhebungen somit einen punktuell höheren Druck auf die zu behandelnde Stelle übertragen. Die Beweglichkeit der Erhebungen wird durch die härtere Ausbildung jedoch reduziert.

Gemäß einer zweiten Ausführungsform der Erfindung ist die Erhebung, insbesondere das Pelottendruckelement aus einem Material gefertigt, das weicher ist als das Material des Grundkörpers, sodass es eine höhere Elastizität und eine reduzierte Steifigkeit im Vergleich zu dem Grundkörper aufweist. Dadurch wird den Druckvorsprüngen eine hohe Beweglichkeit in sich geboten, die insbesondere eine chaotische Bewegung von insbesondere mehreren Druckvorsprüngen des Pelottendruckelements bewirkt.

In einer bevorzugten Ausführungsform hat das Material der Erhebung, insbesondere des Pelottendruckelements eine Shorehärte von mindestens 40 Shore 00, bevorzugt mindestens 6 Shore A und höchstens 20 Shore A. In einer bevorzugten Ausführungsform hat das Material der Erhebung, insbesondere des Pelottendruckelements eine Shorehärte von mindestens 20 Shore A und höchstens 80 Shore A. In einer bevorzugten Ausführungsform weist das Material der Erhebung, insbesondere des mindestens einen Pelottendruckelements eine Härte von mindestens 20 Shore A und höchstens 60 Shore A auf. In einer bevorzugten Ausführungsform weist das Material der Erhebung, insbesondere des mindestens einen Pelottendruckelements eine Härte von mindestens 20 Shore A, weiter bevorzugt von mindestens 25 Shore A auf. In einer bevorzugten Ausführungsform weist das Material der Erhebung, insbesondere des mindestens einen Pelottendruckelements eine Härte von höchstens 50 Shore A, auf. In einer bevorzugten Ausführungsform weist das Material des der Erhebung, insbesondere mindestens einen Pelottendruckelements eine Härte von höchstens 49 Shore A, weiter bevorzugt von höchstens 45 Shore A auf. Bevorzugt weist das Material der Erhebung, insbesondere des mindestens einen Pelottendruckelements eine Härte von mindestens 35 bis höchsten 45 Shore A auf. Bevorzugt weist das Material der Erhebung, insbesondere des mindestens einen Pelottendruckelements eine Härte von etwa 40 Shore A auf.

In einer bevorzugten Ausführungsform hat das Material des Pelottengrundkörpers eine Shorehärte von mindestens 20 Shore 00 und höchstens 60 Shore 00 und/oder das Material der Erhebung, insbesondere des Pelottendruckelements hat eine Shorehärte von mindestens 40 Shore 00, bevorzugt mindestens 6 Shore A und höchstens 20 Shore A.

Vorzugsweise ist die mindestens eine Erhebung noppenförmig, zapfenförmig, zylinderförmig, pyramidenförmig, kegelförmig und/oder pilzförmig ausgebildet. Je nach Anwendungsziel bietet sich hier eine andere Form an und bei mehreren Erhebungen können diese auch eine unterschiedliche Form haben. Durch die Erhebungen und die erfindungsgemäße Ausbildung der Pelotte wird erreicht, dass die beweglichen Erhebungen zu einem Wechseldruck, wie ihn zum Beispiel auch ein Therapeut bei einer Massage ausgeübt, damit die Druckwirkung auf sensible Nerven, Lymph- und Blutgefäße lange Zeit bestehen bleibt. Dies ist insbesondere vorteilhaft, wenn die Pelotte als Kniepelotte ausgebildet ist, und dort ein entsprechender Massageeffekt bei der Meniskusbasis und den hoffaschen Fettkörpern ansetzt.

Vorzugsweise ist die eine oder zumindest eine der mehreren Erhebungen in ihrer Längserstreckung senkrecht zur Außenfläche des Pelottengrundkörpers oder geneigt zur Außenfläche des Grundkörpers ausgerichtet. Durch eine geneigte Ausrichtung der Erhebung zur Außenfläche wird der Erhebung bei der Behandlung eine Verformungsrichtung bereits vorgegeben. Wird entgegen der Neigung die Erhebung belastet, so wirkt sie besonders steif und hart, wird er in Neigungsrichtung belastet, so weicht sie umso schneller oder mit umso geringerem Kraftaufwand zurück. Dadurch kann je nach Anwendungsfall die Druckverteilung bei der Anwendung der Pelotte optimal auf den jeweiligen Patienten eingestellt werden. Erstreckt sich die Erhebung senkrecht zur Außenfläche des Grundkörpers, so ist er in alle Richtungen quer zu ihrer Längserstreckung gleichermaßen beziehungsweise mit gleicher Kraft verformbar.

In einer bevorzugten Ausführungsform, insbesondere als Patellapelotte, weist der Pelottengrundkörper mindestens einen, bevorzugt zwei Flügel auf, wobei der mindestens eine Flügel mindestens eine Erhebung, bevorzugt Noppen und/oder Stege, aufweist.

Vorzugsweise ist die Pelotte eine Kniepelotte, eine Sprunggelenkpelotte, eine Rückenpelotte, eine Ellbogenpelotte, eine Pelotte für den Armbereich oder eine Pelotte für den Bauchbereich.

Bevorzugt handelt es sich bei der erfindungsgemäßen Pelotte um eine Pelotte für die Kniescheibe, eine Pelotte für den Ellenbogen oder eine Pelotte für das Handgelenk handelt. Bevorzugt handelt es sich bei der erfindungsgemäßen Pelotte um eine Pelotte für die Kniescheibe, also eine Patellapelotte.

In einer bevorzugten Ausführungsform weist der Pelottengrundkörper in der Vorderseite für die wenigstens eine Erhebung, insbesondere das wenigstens eine Pelottendruckelement eine Vertiefung auf, deren Kontur der Kontur der mindestens einen Erhebung, bevorzugt des Pelottendruckelements zumindest im Wesentlichen derart entspricht, dass zwischen dem Grundkörper und der Erhebung, bevorzugt dem Pelottendruckelement zumindest abschnittsweise in Umfangsrichtung der Erhebung, bevorzugt des Pelottendruckelements gesehen eine Nut ausgebildet ist. Bei dieser Nut handelt es sich um eine Abstandsnut, also nicht um die Dehnungsnut. Diese Abstandsnut ist also eine zusätzliche Nut zu der mindestens einen Dehnungsnut. Dies hat den Vorteil, dass durch eine konstruktiv einfach umsetzbare Anpassung die Beweglichkeit des oder der Erhebungen erhöht und damit die Massagewirkung bei weicheren, härteren und/oder kleineren Erhebungen zumindest gleichbleibt oder verbessert wird. Durch die Nut wird ein seitlicher Abstand zwischen Grundkörper und Pelottendruckelement definiert. Damit ist das Pelottendruckelement und insbesondere die daran angeordnete Erhebung nicht in direktem seitlichem Kontakt mit dem Grundkörper. Das zumindest eine Pelottendruckelement ist somit in dem Grundkörper schwimmend gelagert. Dadurch ergibt sich, dass bei einer Anwendung und Belastung der Druckvorsprung durch den Grundkörper weniger stark gestützt wird als bisher und dadurch leichter verformbar ist. Dadurch kann die Pelotte in ihrer Höhe, also Bezug auf die Höhenerstreckung oder Längserstreckung der Druckvorsprünge, bei gleichbleibender Massagewirkung kleiner dimensioniert werden. Insbesondere ist vorgesehen, dass der jeweilige Druckvorsprung seitlich bündig mit dem Grundelement abschließt, also direkt an der Außenkante des Grundelements, die die Außenkontur des Pelottendruckelement definiert, liegt. Damit wirkt die Nut direkt mit dem jeweiligen Pelottendruckelement zusammen beziehungsweise verhindert, dass der jeweilige Pelottendruckelement seitlich direkt durch das Material des Grundkörpers gestützt ist.

Gemäß einer bevorzugten Ausführungsform der Erfindung erstreckt sich die Nut über den gesamten Umfang der Erhebung, insbesondere des Pelottendruckelements. Damit ist unabhängig von der Ausgestaltung der Erhebung deren Beweglichkeit in der oben beschriebenen vorteilhaften Art und Weise gewährleistet. Durch die Ausbildung einer Umfangsnut entspricht die Kontur der Vertiefung der Kontur des Pelottendruckelements beziehungsweise des Grundelements, wodurch außerdem auch die Fertigung der Pelotte vereinfacht ist.

Vorzugsweise weist die Abstandsnut eine Breite, also einen Abstand von dem Grundelement zu dem Grundkörper, von 0,3 Millimeter. Es hat sich gezeigt, dass hierdurch die oben genannten Eigenschaften in besonders vorteilhafter Weise erreicht werden.

Gemäß einer bevorzugten Weiterbildung der Erfindung weist die Nut, also Abstandsnut in ihrer Längserstreckung eine konstante Tiefe oder zumindest zwei unterschiedliche Tiefen auf. Durch die Tiefe der Nut wird der Höhenbereich, in welchem Grundkörper und Pelottendruckelement beabstandet zueinander liegen, verändert beziehungsweise beeinflusst. Mit abnehmender Tiefe der Nut wirkt der Grundkörper mit höherer Stützwirkung auf das Grundelement und den jeweiligen Druckvorsprung. Mit zunehmender Tiefe nimmt die Beeinflussung und damit die Versteifung des Pelottendruckelements ab. So kann sowohl durch die Breite als auch durch die Tiefe der Nut, in Umfangsrichtung des Grundkörpers gesehen, die Zielfunktion des Pelottendruckelements eingestellt beziehungsweise angepasst werden.

Gemäß einer bevorzugten Ausführungsform der Erfindung weist die Nut, also Abstandsnut eine Tiefe auf, die mindestens 10% der Höhe des Grundkörpers am Rand der Nut entspricht. Die Nut kann vergleichbar einer "Schallplattenrille" ausgestaltet sein. Somit kann diese in Ihrer Breite und ihrer Tiefe variabel ausgeprägt sein.

Gemäß einer bevorzugten Ausführungsform der Erfindung sind das Pelottendruckelement und der Grundkörper aus dem gleichen Material gefertigt. Hierbei ergibt sich die vorteilhafte Beweglichkeit der Druckvorsprünge durch die Abstandsnut. Durch den Einsatz des gleichen Materials wird der Herstellungsaufwand jedoch reduziert. Vorzugsweise sind dann außerdem Grundkörper und Grundelement einstückig miteinander ausgebildet, sodass es sich um einen EinKomponenten-Körper handelt. Hierdurch ist eine besonders zeit- und kostensparende Herstellung der Pelotte insgesamt gewährleistet. Die Beweglichkeit der Druckvorsprünge beziehungsweise des Pelottendruckelements wird durch die Wahl einer passenden Nutbreite und/oder Nuttiefe der Abstandsnut dann individuell an den Bedarf angepasst.

Die vorliegende Erfindung betrifft auch ein orthopädisches Hilfsmittel, beispielsweise eine Bandage oder Orthese, aufweisend eine erfindungsgemäße Pelotte. Die vorliegende Erfindung betrifft insbesondere auch eine Bandage oder Orthese, aufweisend eine erfindungsgemäße Pelotte, beispielsweise eine Kniebandage oder -orthese, eine Sprunggelenkbandage oder -orthesee, eine Rückenbandage oder -orthese, eine Ellbogenbandage oder -orthese, eine Bandage oder Orthese für den Armbereich oder eine Bandage oder Orthese für den Bauchbereich.

Bevorzugt handelt es sich um eine Kniegelenksbandage oder-orthese, einen Ellenbogenbandage oder -orthese oder eine Handgelenksbandage oder -orthese. Bevorzugt handelt es sich um eine Kniegelenksbandage oder-orthese. Bevorzugt handelt es sich um eine Kniegelenksbandage.

Die erfindungsgemäße Orthese oder Bandage zeichnet sich durch die erfindungsgemäße Pelotte aus. Es ergeben sich dadurch die oben bereits genannten Vorteile. Vorzugsweise ist die Orthese eine elastische Gestrickorthese oder die Bandage eine elastische Gestrickbandage, insbesondere Knieorthese oder Kniebandage.

Weitere Vorteile und bevorzugte Merkmale und Merkmalskombinationen ergeben sich aus dem zuvor Beschriebenen sowie aus den Ansprüchen. Im Folgenden soll die Erfindung anhand der Zeichnungen näher erläutert werden. Dazu zeigen
- Figur 1: eine vorteilhafte Pelotte in einer perspektivischen Draufsicht,
- Figur 2: Einen Ausschnitt eines Dehnungsbereichs der Pelotte aus Figur 1,
- Figur 3: Einen Ausschnitt einer bevorzugten Ausführungsform einer erfindungsgemäßen Pelotte.

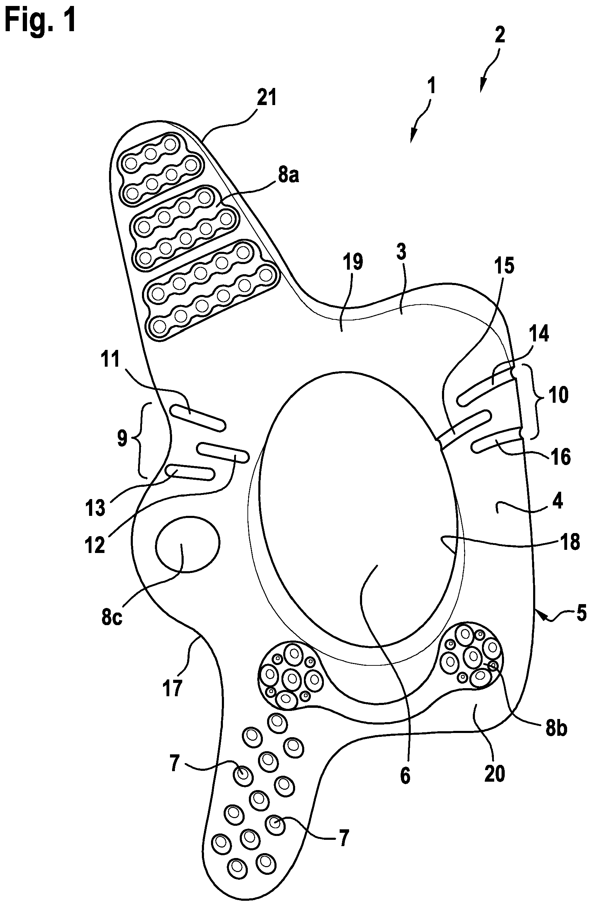

**Figur 1** zeigt in einer Darstellung eine vorteilhafte Pelotte 1 für eine hier nicht näher dargestellten Orthese oder Bandage 2. Bei der gezeigten Ausführungsform der Pelotte handelt es sich um eine Patellapelotte. Die Pelotte 1 weist einen Pelottengrundkörper 3 auf, der im Wesentlichen flächig ausgebildet ist und aus einem elastischen, insbesondere viskoelastischen Material gefertigt ist. Der Grundkörper 3 weist eine dreidimensionale Außenkontur auf mit einer Vorderseite 4 als Behandlungsseite und mit einer von der Vorderseite 4 abgewandten Rückseite 5. Gemäß dem vorliegenden Ausführungsbeispiel ist die Außenkontur des Grundkörpers 3 durch mehrere Flügel gekennzeichnet, die von einem Basisring insbesondere radial nach außen vorstehen, wobei in dem Basisring außerdem eine oval-förmige Öffnung als Aussparung 6 ausgebildet ist. An den Flügeln und am Basisring weist die Pelotte mehrere Erhebungen, also Pelottendruckelemente verschiedener Formen auf, wobei manche Pelottendruckelemente 7 aus demselben Material wie der Grundkörper gebildet sind und manche Pelottendruckelemente 8a, 8b, 8c aus einem härteren Material.

Die Pelotte weist erfindungsgemäß zwei paarig angeordnete Dehnungsbereiche 9,10 auf, die jeweils durch drei als in den Grundkörper 3 eingeschnittene Nuten 11,12,13,14,15,16 ausgebildet sind, die als Dehnungsnuten fungieren. Dabei reicht keine der Dehnungsnuten 11,12,13,14,15,16 von der Außenseite 17 des Pelottengrundkörpers 3 zu der zur Aussparung 6 hin gerichteten Innenseite 18 des Pelottengrundkörpers. Durch die paarig angeordneten Dehnungsbereiche 9,10 wird der Pelottengrundkörper 3 in einen ersten Pelottengrundkörperbereich 19 und einen zweiten Pelottengrundkörperbereich 20 geteilt, wobei hier bei der Patellapelotte der erste Pelottengrundkörperbereich 19 oben positioniert ist und der zweite Pelottengrundkörperbereich 20 unten positioniert ist.

Durch die paarig angeordneten Dehnungsbereiche 9,10 können sich der obere, erste Pelottengrundkörperbereich 19 und der untere, zweite Pelottengrundkörperbereich 20 bei einer Beugung des Knies wesentlich stärker voneinander wegbewegen, als dies bei einer Pelotte ohne die Dehnungsbereiche der Fall wäre. Auf einer Uhr können die Positionen der Dehnungsbereiche 9,10 "auf 2 Uhr und 10 Uhr" beschrieben werden. Es sind natürlich aber auch andere Positionen möglich, beispielsweise "auf 3 Uhr und 9 Uhr" "auf 4 Uhr und 8 Uhr". Wesentliches Merkmal ist aber, dass alle Dehnungsbereiche nur paarweise eingesetzt die gewünschte Funktion ausüben.

Eine derartige Dehnbarkeit wäre alternativ nur über ein weicheres Grundmaterial der Pelotte selbst zu erreichen. Damit wären die Funktionen der Pelotte also Positionssicherheit der Kniescheibe und der gesamten Kniebandage, Abstandshalter für das die Kniescheibe überspannende elastische Gestrick, aber nicht mehr im geforderten Maße möglich. Idealerweise sind die Dehnungsbereiche, insbesondere Dehnungsnuten rechts und links vom Gelenksspalt des Kniegelenks angebracht. Denn bei einer Beugung des Knies muss der obere Teil 19 der Pelotte in der Lage sein, seine Position gegenüber dem oberen Ende der Kniescheibe beizubehalten. Nur dann ist sichergestellt, dass auf dem oberen Bereich der Pelotte 1 liegende Erhebungen, insbesondere Pelottendruckelemente 8a ihre Position beibehalten. Bei Pelotten aus dem Stand der Technik wird bei Verwendung der oberhalb des Pelotten-Ovals liegende vernoppte Bereich oft so stark verschoben, dass die auf ihm befindlichen Noppen "umknicken", anstelle wie vorgesehen senkrecht zum zur Körperoberfläche Massagedruck einzutragen. Diese senkrechte Wirkung der Noppen ist allerdings als gezielte Nachahmung für die therapeutische Massagen auf den Hauptmuskel "Musculus vastus medialis" vorteilhaft. Kippen aber die in diesem Bereich befindlichen Noppen weg, fällt die sensomotorische Antwort des Muskels auf den Stimulus nicht wie erforderlich aus. Die erfindungsgemäßen paarigen Dehnungsbereiche 9,10 stabilisieren in vorteilhafter Weise die Position der beiden Pelottengrundkörperbereiche.

In der gezeigten Patellapelotte 1 ist über dem Hauptmuskel "vastus medialis" ein mit Pelottendruckelementen 8a versehener seitlicher Flügel 21 mitgegossen, welcher bei Druck auf den Muskel eine Massage mit Lösung der Kontrakturen und eine sensomotorische Antwort des Muskels erzeugt.

Die erfindungsgemäße Kombination der Dehnungsbereiche 9,10 mit den über dem Musculus vastus medialis eingearbeiteten intermittiernd massierenden Pelottendruckelementen 8a erzeugt die gewünschte Schmerzlinderung durch Entspannung des beim Arthrosepatienten schmerzhaft kontrakten Muskels insbesondere auch durch die positive Beeinflussung der Triggerpunkte. Dies entspricht der schmerzlindernden Wirkung, welche der Physiotherapeut mit einer manuellen Friktionsmassage erzeugt. Weitere schmerzlindernde Vorteile sind die verringerte Zugbelastung auf das Ligamentum patellae, die verringerte Schubbelastung der arthrotischen Patella und die geringere Rutschgefahr der Bandagen.

Die überraschenden Vorteile einer Kombination von Dehnungsbereichen 9,10 und Pelottendruckelementen 8a kann der Fachmann auf andere Pelotten wie Ellenbogenpelotten oder Handgelenkspelotten übertragen.

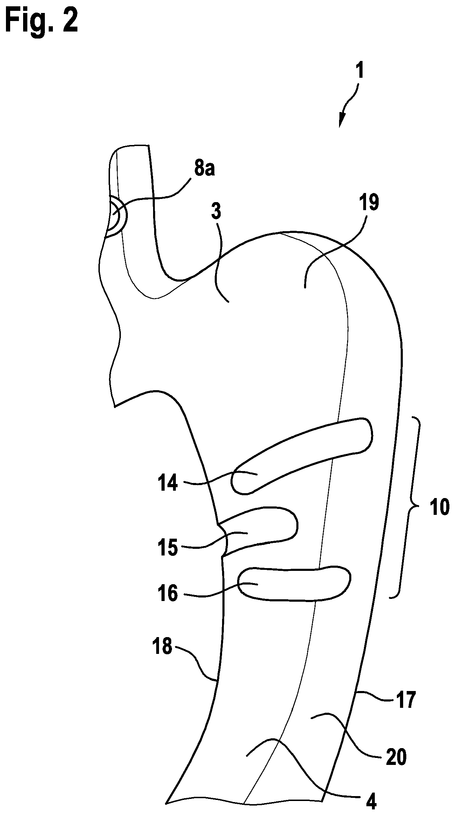

Zur Verdeutlichung der Dehnungsbereiche ist in **Figur 2** ein vergrößerter Ausschnitt der Pelotte 1 zu sehen. Der Dehnungsbereich 10 besteht aus den drei Dehnungsnuten 14,15,16, die auf der Vorderseite 4 des Pelottengrundkörpers 3 eingebracht sind. Die Dehnungsnuten sind alle nicht durchgängig, bilden also keinen Kanal zwischen der zur Aussparung hin gerichteten Innenseite 18 des Pelottengrundkörpers 3 und der Außenseite 17 des Pelottengrundkörpers 3, sondern die Nuten 14 und 16 sind nur zur Außenseite 17 des Pelottengrundkörpers 3 hin geöffnet und die Nut 15 nur zur Aussparung hin gerichteten Innenseite 18 des Pelottengrundkörpers 3. Der Dehnungsbereich 10 teilt den Pelottengrundkörpers 3 in die zwei Pelottengrundkörperbereiche 19,20. Der obere, erste Pelottengrundkörperbereich 19 weist die Pelottendruckelemente 8a auf, die durch die paarigen Dehnungsbereiche 10, 11 bei Bewegung besser positioniert bleiben.

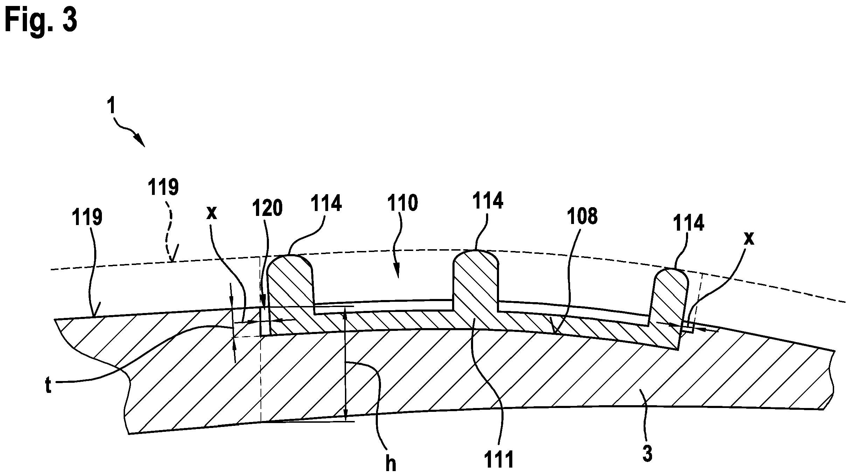

Zur Verdeutlichung der Pelottendruckelemente mit Abstandsnut ist in **Figur 3** eine Längsschnittdarstellung der Pelotte 1 im Bereich des Pelottendruckelements 8b, hier als 110 bezeichnet, gemäß **Figur 1** gezeigt.

Die Vertiefung 108 weist also eine Tiefe auf, die gemäß dem vorliegenden Ausführungsbeispiel mindestens so groß ist wie die Höhe des eingesetzten Grundelements 111 des Pelottendruckelements 110. Die Druckvorsprünge 114 sind derart groß in ihrer Höhe ausgebildet, dass sie aus der Vertiefung heraus von dem Grundkörper 3 der Pelotte 1 vorstehen. Während gemäß dem vorliegenden Ausführungsbeispiel die Vertiefung 108 einen geraden oder ebenen Boden aufweist, ist gemäße einem weiteren Ausführungsbeispiel - hier nicht gezeigt - vorgesehen, dass der Boden der Vertiefung von einer geraden Ebene abweicht. Dadurch kann das Verhalten der Druckvorsprünge 114 im Gebrauch weiter beeinflusst und beispielsweise eine Vorzugsbiegerichtung vorgegeben werden.

Die Kontur oder Innenkontur der jeweiligen Vertiefung 108 entspricht dabei zumindest im Wesentlichen der Kontur oder Außenkontur des jeweiligen Pelottendruckelements 110, insbesondere der des jeweiligen Grundelements 111. Dabei ist die Kontur der jeweiligen Vertiefung 108 derart gewählt, dass zumindest abschnittsweise in Umfangsrichtung des jeweiligen Grundelements 111 gesehen ein seitlicher Abstand x verbleibt. Durch den Abstand x entsteht zwischen dem jeweiligen Pelottendruckelement 110 und dem Grundkörper 3 eine Nut, also Abstandsnut 120. Diese Abstandsnut 120 erstreckt sich zumindest abschnittsweise entlang des Umfangs des jeweiligen Pelottendruckelements. Der Abstand x, der durch die Nut 120 gebildet wird, beträgt vorzugsweise 0,1 mm bis 3 m, vorzugsweise 0,5 mm bis 1,5 mm.

Gemäß einem vorteilhaften Ausführungsbeispiel erstreckt sich die Abstandsnut 120 über den gesamten Umfang des Pelottendruckelements 110. In ihrer Längserstreckung weist die Nut 120 bevorzugt eine konstante Tiefe t auf.

Die Nut 120 weist vorzugsweise eine Tiefe t auf, die mindestens 10% der Höhe h des Grundkörpers 3 am Rand der Nut 120 entspricht.

Durch die Abstandsnut 120 wird erreicht, dass das Einfederungsverhalten und auch eine Einfederungsrichtung des Druckvorsprungs 114 vorteilhaft beeinflusst wird.

Die Druckvorsprünge 114, die vorliegend in Form von Noppen mit gerundeten Enden ausgebildet sind, sind bevorzugt aus einem festeren Material als der Grundkörper 3 ausgebildet. Bei Pelotten, deren Druckvorsprünge 114 härter ausgestaltet sind als der Grundkörper 3 ist der Massageeffekt, der durch die Pelotte 1 beziehungsweise eine Pelotte 1 aufweisenden Orthese oder Bandage 2 erreicht werden kann, höher.

Die Tiefe t und Breite x der Nut 120, die den jeweiligen Druckvorsprung oder das Grundelement 111 umgibt, bestimmt die Beweglichkeit des jeweiligen zugeordneten Druckvorsprungs 114. Weil das sich unmittelbar an den jeweiligen Druckvorsprung 114 anschließende Material des Grundkörpers 3 dünner ist beziehungsweise entfernt ist, kann der einzelne Druckvorsprung 114 wegen des aus der Nut 120 resultierenden Abstands x besser einfedern und auch generell innerhalb des ausgedehnten Randbereichs sich besser bewegen. Dies gilt unabhängig davon, ob der jeweilige Druckvorsprung 114 vollständig in die Vertiefung 108 versenkt ist, und beispielsweise mit der Außenfläche 119 des Grundkörpers 3 bündig abschließt, oder ob der jeweilige Druckvorsprung 114 wie in den vorliegenden Ausführungsbeispielen gezeigt, von der Außenfläche 119 vorsteht.

Die Nut 120 hilft dabei, das Pelottendruckelement 110 insgesamt kleiner ausfallen zu lassen, als dies bei bündig abschließender Lagerung auf einer ebenen Lagerungsebene ohne Nut der Fall wäre. Die Pelottendruckelemente 110 sind im Bereich der Nut 120 somit schwimmend gelagert.

Für bestimmte Anwendungen kann es vorteilhaft sein, wenn die Druckvorsprünge beziehungsweise die Pelottendruckelemente 110 nicht oder nur geringfügig von dem Grundkörper 3 hervortreten, jedoch an ihrer jeweiligen Position insbesondere in Bezug auf ihre Druckvorsprünge 114 sehr beweglich sind. Gemäß einem weiteren Ausführungsbeispiel enden somit den Druckvorsprünge 114 insbesondere bündig mit der Außenfläche 119 wie in **Figur 3** beispielhaft durch eine gestrichelte Linie der Außenfläche gezeigt.

### BEZUGSZEICHENLISTE

- 1: Pelotte
- 2: Orthese oder Bandage
- 3: Pelottengrundkörper
- 4: Vorderseite
- 5: Rückseite
- 6: Aussparung
- 7: Erhebungen/ Pelottendruckelemente
- 8: a bis c Pelottendruckelemente
- 9: erster Dehnungsbereich
- 10: zweiter Dehnungsbereich
- 11: Dehnungsnut
- 12: Dehnungsnut
- 13: Dehnungsnut
- 14: Dehnungsnut
- 15: Dehnungsnut
- 16: Dehnungsnut
- 17: Außenseite des Pelottengrundkörpers
- 18: zur Aussparung hin gerichteten Innenseite des Pelottengrundkörpers
- 19: erster Pelottengrundkörperbereich
- 20: zweiter Pelottengrundkörperbereich
- 21: oberer Pelottenflügel

- 108: Vertiefung
- 110: Pelottendruckelemente
- 111: Grundelement des Pelottendruckelements
- 114: Druckvorsprünge
- 119: Außenfläche
- 120: Abstandsnut
- h: Höhe des Grundkörpers
- t: Tiefe
- x: Abstand

## Patentansprüche

1. Pelotte (1), aufweisend einen Pelottengrundkörper (3), wobei der Pelottengrundkörper (3) eine Vorderseite (4) und eine Rückseite (5) aufweist, wobei die Vorderseite (4) des Pelottengrundkörpers (3) mindestens eine Erhebung (7) aufweist, wobei der Pelottengrundkörper (3) mindestens zwei paarig angeordnete Dehnungsbereiche (9,10) aufweist, wobei die zwei Dehnungsbereiche (9,10) den Pelottengrundkörper (3) in einen ersten Pelottengrundkörperbereich (19) und einen zweiten Pelottengrundkörperbereich (20) aufteilen und wobei es sich bei den mindestens zwei Dehnungsbereichen (9,10) jeweils um mindestens eine Nut (11,12,13,14,15,16) im Pelottengrundkörper (3) handelt, wobei der Pelottengrundkörper (3) eine Aussparung (6) aufweist, wobei der Pelottengrundkörper (3) eine Außenseite (17) aufweist und eine zur Aussparung (6) hin gerichtete Innenseite (18) aufweist, **dadurch gekennzeichnet, dass** die mindestens eine Nut (11,12,13,14,15,16) an der Außenseite (17) beginnt und nicht bis zur Innenseite (18) reicht oder an der Innenseite (18) beginnt und nicht bis zur Außenseite (17) reicht.

2. Pelotte nach Anspruch 1, wobei der Pelottengrundkörper (3) mindestens zwei Dehnungsbereiche (9,10) aufweist, die im Pelottengrundkörper (3) durch die Aussparung (6) getrennt werden.

3. Pelotte nach einem der vorhergehenden Ansprüche mit exakt zwei Dehnungsbereichen (9,10).

4. Pelotte nach einem der vorhergehenden Ansprüche, wobei die mindestens eine Nut (11,12,13,14,15,16) sich auf der Vorderseite (4) des Pelottengrundkörpers (3) befindet.

5. Pelotte nach einem der vorhergehenden Ansprüche, wobei die Aussparung (6) einen Durchmesser von mindestens 3 cm aufweist.

6. Pelotte nach einem der vorhergehenden Ansprüche, wobei es sich bei der mindestens einen Erhebung (7) um Noppen und/oder Stege handelt.

7. Pelotte nach einem der vorhergehenden Ansprüche, wobei die Erhebungen (7) härter sind als der Pelottengrundkörper (3).

8. Pelotte nach einem der Ansprüche 1 bis 6, wobei die Erhebungen (7) weicher sind als der Pelottengrundkörper (3).

9. Pelotte nach einem der vorhergehenden Ansprüche, wobei der Pelottengrundkörper (3) mindestens einen, bevorzugt zwei Flügel (21) aufweist, wobei der mindestens eine Flügel mindestens eine Erhebung (7), bevorzugt Noppen und/oder Stege, aufweist.

10. Pelotte nach einem der vorhergehenden Ansprüche, wobei es sich um eine Pelotte (1) für die Kniescheibe, eine Pelotte (1) für den Ellenbogen oder eine Pelotte (1) für das Handgelenk handelt.

11. Bandage oder Orthese (2), aufweisend eine Pelotte (1) nach einem der vorhergehenden Ansprüche.

12. Bandage oder Orthese nach Anspruch 11, wobei es sich um eine Kniegelenksbandage oder-orthese, einen Ellenbogenbandage oder -orthese oder eine Handgelenksbandage oder -orthese handelt.

## Claims

1. Pad (1) comprising a pad base (3), wherein the pad base (3) has a front side (4) and a back side (5), wherein the front side (4) of the pad base (3) has at least one projection (7), wherein the pad base (3) has at least two stretch areas (9, 10) arranged in pairs, wherein the two stretch areas (9, 10) divide the pad base (3) into a first pad base area (19) and a second pad base area (20), and wherein the at least two stretch areas (9, 10) each are at least one groove (11, 12, 13, 14, 15, 16) in the pad base (3), wherein the pad base (3) comprises a recess (6), wherein the pad base (3) comprises an outer side (17) and an inner side (18) facing the recess (6), **characterised in that** the at least one groove (11, 12, 13, 14, 15, 16) begins at the outer side (17) and does not extend as far as the inner side (18), or begins at the inner side (18) and does not extend as far as the outer side (17).

2. Pad according to claim 1, wherein the pad base (3) comprises at least two stretch areas (9, 10) which are separated within the pad base (3) by the recess (6).

3. Pad according to any of the preceding claims, comprising exactly two stretch areas (9, 10).

4. Pad according to any of the preceding claims, wherein the at least one groove (11, 12, 13, 14, 15, 16) is located on the front side (4) of the pad body (3).

5. Pad according to any of the preceding claims, wherein the recess (6) has a diameter of at least 3 cm.

6. Pad according to any of the preceding claims, wherein the at least one projection (7) are nubs and/or ridges.

7. Pad according to any of the preceding claims, wherein the projections (7) are harder than the pad base (3).

8. Pad according to any of claims 1 to 6, wherein the projections (7) are softer than the pad base (3).

9. Pad according to any of the preceding claims, wherein the pad base (3) comprises at least one, preferably two, wings (21), wherein the at least one wing comprises at least one projection (7), preferably nubs and/or ridges.

10. Pad according to any of the preceding claims, wherein it is a pad (1) for the kneecap, a pad (1) for the elbow or a pad (1) for the wrist.

11. Bandage or orthosis (2) comprising a pad (1) according to any of the preceding claims.

12. Bandage or orthosis according to claim 11, wherein it is a knee joint bandage or orthosis, an elbow bandage or orthosis, or a wrist bandage or orthosis.

## Revendications

1. Pelote (1) ayant un corps de base de pelote (3), le corps de base de pelote (3) ayant une face avant (4) et une face arrière (5), la face avant (4) du corps de base de pelote (3) ayant au moins une élévation (7), le corps de base de pelote (3) ayant au moins deux zones d'étirement disposées par paires (9,10), les deux zones d'étirement (9,10) divisant le corps de base de pelote (3) en une première zone de corps de base de pelote (19) et une deuxième zone de corps de base de pelote (20), et les au moins deux zones d'étirement (9,10) étant chacune au moins une rainure (11,12,13,14,15,16) dans le corps de base de pelote (3), le corps de base de pelote (3) ayant un évidement (6), le corps de base de pelote (3) ayant une face extérieure (17) et une face intérieure (18) orientée vers l'évidement (6), **caractérisé en ce que** l'au moins une rainure (11,12,13,14,15,16) commence au niveau de la face extérieure (17) et ne s'étend pas jusqu'à la face intérieure (18) ou commence au niveau de la face intérieure (18) et ne s'étend pas jusqu'à la face extérieure (17).

2. Pelote selon la revendication 1, dans laquelle le corps de base de pelote (3) présente au moins deux zones d'étirement (9,10) qui sont séparées dans le corps de base de pelote (3) par l'évidement (6).

3. Pelote selon l'une quelconque des revendications précédentes avec exactement deux zones d'étirement (9,10).

4. Pelote selon l'une quelconque des revendications précédentes, dans laquelle l'au moins une rainure (11,12,13,14,15,16) se trouve sur la face avant (4) du corps de base de pelote (3).

5. Pelote selon l'une quelconque des revendications précédentes, dans laquelle l'évidement (6) a un diamètre d'au moins 3 cm.

6. Pelote selon l'une quelconque des revendications précédentes, dans laquelle l'au moins une élévation (7) est constituée de picots et/ou de nervures.

7. Pelote selon l'une quelconque des revendications précédentes, dans laquelle les élévations (7) sont plus dures que le corps de base de pelote (3).

8. Pelote selon l'une quelconque des revendications 1 à 6, dans laquelle les élévations (7) sont plus molles que le corps de base de pelote (3).

9. Pelote selon l'une quelconque des revendications précédentes, dans laquelle le corps de base de pelote (3) a au moins une aile, de préférence deux ailes (21), dans laquelle l'au moins une aile a au moins une élévation (7), de préférence des picots et/ou des nervures.

10. Pelote selon l'une quelconque des revendications précédentes, dans laquelle il s'agit d'une pelote (1) pour la rotule, d'une pelote (1) pour le coude ou d'une pelote (1) pour le poignet.

11. Bandage ou orthèse (2), ayant une pelote (1) selon l'une quelconque des revendications précédentes.

12. Bandage ou orthèse selon la revendication 11, dans lequel il s'agit d'un bandage ou d'une orthèse du genou, d'un bandage ou d'une orthèse du coude ou d'un bandage ou d'une orthèse du poignet.
